(19) Europäisches Patentamt
European Patent Office
Office européen des brevets

(11) EP 2 106 788 A1

(12) # EUROPEAN PATENT APPLICATION

(43) Date of publication:
**07.10.2009 Bulletin 2009/41**

(21) Application number: **08290330.3**

(22) Date of filing: **04.04.2008**

(51) Int Cl.:
***A61K 9/19** (2006.01)* ***A61K 38/00** (2006.01)*
***A61P 35/00** (2006.01)*

(84) Designated Contracting States:
**AT BE BG CH CY CZ DE DK EE ES FI FR GB GR HR HU IE IS IT LI LT LU LV MC MT NL NO PL PT RO SE SI SK TR**
Designated Extension States:
**AL BA MK RS**

(71) Applicant: **IPSEN PHARMA**
**92100 Boulogne-Billancourt (FR)**

(72) Inventors:
- **Nourisson, Didier**
  **28500 Vernouillet (FR)**
- **Mondoly, Nathalie**
  **78150 Le Chesnay (FR)**
- **Benamer, Naziha**
  **78711 Mantes-La-Ville (FR)**

(74) Representative: **Bourgouin, André**
**BEAUFOUR IPSEN - SCRAS**
**Direction Propriété Intellectuelle**
**24 Rue Erlanger**
**75016 Paris Cedex 16 (FR)**

(54) **Liquid and freeze dried formulations**

(57) The present invention relates to stable parenteral formulations comprising a new class of compounds, which is a non proteinic compound, a method for preparing such formulations as well as the use of certain compounds for stabilizing these formulations.

EP 2 106 788 A1

## Description

The present invention relates to liquid and freeze dried formulations of a new class of compounds, which is a non proteinic compound, a method for preparing such formulations as well as the use of certain compounds for stabilizing these formulations.

The active substance used in the present invention is a chimeric molecule consisting of two dopamine agonist, small molecule moieties covalently linked to the N-terminus of a cyclic peptide somatostatin analogue and acts as a chimeric compound.

Specifically, the invention concerns stable formulations at temperatures that can range from +5° C to +40° C and which can be reconstituted in liquid form by adding a solvent for its administration through parenteral administration.

As further object, the present invention encompasses a stable liquid pharmaceutical formulation at temperatures that can range from +5° C to +40°-C and 60 to 85 % RH (relative humidity) preferably 75 % RH. This liquid formulation may be kept in a vial or as a ready to use formulation in a syringe type device.

It is known that freeze-drying process may have a considerable effect on degradation of the pharmaceutically active substance in the formulation, as well as a strong influence on their stability in freeze dried form or after reconstitution of the suspension or solution.

Difficulties are also encountered for liquid formulations in order to maintain activity, efficiency and ratio of the active substances as well as clarity of the liquid over time.

Various approaches to stabilizes freeze-dried and liquid formulations have been discussed in the literature.

For instance, pharmaceutical compositions in the form of freeze-dried preparation for parenteral injection use are described in the scientific publication "Stabilisation of Octastation, a somatostatin analogue. Preparation of freeze-dried products for parenteral injection", H. Pourrat & Al, Biological and Pharmaceutical Bulletin, Vol. 18, No. 5, pp 766-771, The Pharmaceutical society of Japan. The publication describes a formulation with glutamic acid-sodium glutamate buffer as a stabilizing agent and the freeze-drying procedure found appropriate for subsequent industrial production.

The use of sugars as stabilizing agent is known with proteins in pharmaceutical formulation. In the international patent application WO 0056365, compositions comprising an antigen which consists of a polysaccharide bound to a carrier protein and trehalose are reported.

The interest for novel dopamine-somatostatin chimeric molecules with differing, enhanced activity at specific receptor has been demonstrated in the scientific publication "Efficacy of chimeric molecules directed towards multiple somatostatin and dopamine receptors on inhibition of GH and prolactin secretion from GH-secreting pituitary adenomas classified as partially responsive to somatostatin analog therapy", P Jaquet & Al, European Journal of Endocrinology, Vol 153, Issue 1, pp 135-141. These class of compounds, consistently produced significatly greater suppression of GH (growth hormone) and PRL (prolactin) than either octreotide or single-receptor-interacting ligands in tumors from patients classified as only partially responsive to octreotide therapy.

The problem solved by the present invention is to provide stable formulations over time containing this chimeric compound in freeze-dried form or liquid,form by means of the specific freeze-dried process and/or adequate choice of excipients. The present invention provides a composition which is stable for at least 24 months in liquid or freeze dried form and additionnally a process for freeze drying such compositions.

The following definitions are set forth to illustrate and define the meaning and scope of the various terms used to describe the invention herein.

The terms "lyophilised" "freeze-dried" "lyophilization" as used herein refers to a method of isolating a solid substance from solution by freezing the solution and evaporating the ice under vacuum.

The term "lyophilisate" as used herein refers to a drug substance after freeze-drying process on its own or with different injectable bulking agents.

The term "bulking agents" as used herein consists of a non reducing sugar or a polyhydric alcohol (polyol).

The term "stability" as used in the present invention refers to chemical and physical stability.

The term "powder for solution for infusion" as used herein consists of a powder, such as lyophilisate that can be quickly reconstituted (extemporaneously) in solution with water to be administered by parenteral route.

The term "immediate release formulation" as used herein consists of a release of the active substance for a duration of one day to one week.

The term "amino acid" as used in the present invention denotes an amino acid comprising but not limited to arginin, glycine, lysine histidine, glutamic acid, aspargic acid, isoleucine, leucine, alanine, phenylalanine, tryprophane, serine, methionine, proline the amino acid may used singly or in combination.

The term "pharmaceutically acceptable" means in this context physiologically well-tolerated by a mammal or a human.

The term "high concentrated" in the present invention generally means a content in active substance of 0.1 mg/ml to 20 mg/ml preferably from 1 to 10 mg per 1 vial.

Ther term "buffered isotonic" and "buffered saline" solution refers to a solution containing a salt in addition to

a buffer.

The term "water for injection" as used herein means sterile water obtained after filtration or by known techniques from the skilled person.

Solutol® used in liquid formulation is macrogol 12 Hydroxystearate, a non ionic solubilizer for injection solutions.

Description of figures :

Figure 1a : shows comparative pk profile after single subcutaneous injection in rats at doses 0.2 mg/kg. 2 % solutol® in wfi formulation. Dose in rats corresponding to 0.2 mg/kg. Formulations were prepared according to example 4 (process flow chart, table 6 step A to C).

Figure 1b : shows comparative pk profile after single subcutaneous injection in rats at doses 0.2 mg/kg. Threalose in wfi formulation. Formulations were prepared according to example 4 (process flow chart, table 6 step A to F).

Figure 2 : shows the variation of BIM23A760 in freeze dried form uder storage at 40° C with 75 % HR.

According to the invention, a further object is the use of sugars such as saccharides or disaccharides or polyols or a mixture thereof for the stability of the formulation as well as for storage stability of the liquid or freeze-dried preparation.

Accordingly, saccharides disaccharides and polyols include xylitol, maltose, lactose, galactose, mannitol, sorbitol, dextran and threalose or combination thereof although not limited to these.

Preferred are mannitol, xylitol and threalose even more preferred is threalose.

In a preferred embodiment, the present formulation, freeze dried and/or liquid formulation, comprises between 1 to 50 % by weight of the active substance BIM23A760 (BIM23A760 is Dop2-DLys(Dop2)-c(Cys-Tyr-DTrp-Lys-Abu-Cys)-Thr-$NH_2$) in the formulation or other dopamine derivatives or salt thereof preferably 15 to 30 % by weight of BIM23A760 in the formulation or other dopamine derivatives or salt thereof.

The quantity of polyols, sugars, saccharides or disaccharides according to the formulations of the present invention, comprises about 50 to 95 % by weight of the formulation, preferably the amount of polyol or sugars such as saccharides or disaccharides is between 70 % to 85 % by total weight of the formulation.

The preferred pH range of the formulation is from 3.0 to 7.0, wherein 4.0 to 6.0 is particularly preferred.

Preferably the compositions in the form of freeze-dried product, to be reconstituted by addition of a solvent. The solvent may be an isotonic solution, a buffered isotonic, buffered saline solution or water for injection.

In addition the compositions of the invention may further comprise a conventional buffering agent, such as amino acid or organic salts which include boric acid, phosphoric acid, acetic acid, and citric acid and/or a salt thereof.

The formulations disclose herein may also contains an isotonic agents. A suitable isotonic agent for the formulation according to the invention include propylene glycol, glycerol, sodium chloride, potassium chloride in the amount of 0.1 to 5 % w/w, preferably in the amount of 0.9 to 3 %.

Pharmaceutical salts of active substance usable for compositions according to the invention may be produced by acid addition salts with organic and inorganic acids. Examples of acid addition salts of compounds are salts with mineral acids, for example hydrohalic acids such as hydrochloric acid, hydrogen bromide and hydrogen iodide, sulphuric acid, nitric acid, phosphoric acid and the like, salts with organic sulphonic acids, for example with alkyl- and arylsulphonic acids such as methanesulphonic acid, p-toluenesulphonic acid, benzenesulphonic acid and the like, as well as salts with organic carboxylic acids, for example with acetic acid, tartaric acid, maleic acid, citric acid, benzoic acid, fumaric acid, oxalic acid, stearic acid, salicylic acid, ascorbic acid or insoluble salts such as pamoic acid and the like.

Whether the active substance contains a carboxyl group also form pharmaceutically acceptable salts with bases. Examples of such salts are alkali metal salts, for example sodium and potassium salts, ammonium salts, salts with organic bases, for example with amines such as diisopropylamine, benzylamine, dibenzylamine, triethanolamine, triethylamine, N,N-dibenzylethylenediamine, N-methylmorpholine, pyridine, piperazine, N-ethylpiperidine, N-methyl-D-glucamine and procaine, or with amino acids such as arginine and lysine.

Preferred peptide salt is a salt formed with an organic acid.

The object of the present invention is to provide a freeze-dried and liquid formulation form dopamine derivatives showing an increased stability during manufacture of the formulation and/or during subsequent storage therefore as further object the invention also encompasses a process.

Methods of preparing the compound of the present invention are illustrated in illustrative purposes and should not be constructed as limiting the invention disclosed.

The process according to the invention for the preparation of the liquid or freeze dried formulation may be

carried out as follows :

a) introduction and weighing of the active substance and excipients ;

b) dissolution of compounds by means of water under stirring ;

c) use of Nitrogen for solution bubbling ;

d) filtration ;

e) filling into a vial and pre-stoppering or stoppering under nitrogen for the solution form ;

f) freeze-drying step under vacuum ;

g) releasing vacuum and stoppering under nitrogen.

In accordance with the invention the pharmaceutical formulations are suitably prepared :

- By introducing an appropriate amount of the active drug and excipients.
- Dissolving under stirring, optionally water is used for dissolving the active substance and excipients and may be water for injection, buffered solution or isotonic solution The solution containing a predeterminated concentration of the active substance and excipients may be bubbled under nitrogen. Thereafter the solution may be sterilized by sterile filtration through PVDF (polyvinylidene fluoride) membrane filters and thereafter optionally aseptically filled into sterile vials.
- Optionally stoppering vials under bubbling with nitrogen at room temperature for the liquid form in order to maintain a protected atmosphere and prevent oxidation.
- A further step consisting of pre stoppering vials at room temperature and then pre frozen step may be carried out. The pre frozen temperature could be from about -20° C to -50° C preferably from about -20° C to -30° C at even more preferred at about -25° C to -30° C under inert conditions.
- Subsequently the solution may be frozen, dried optionally under nitrogen atmosphere or other controlled atmostphere known in the art, by primary drying under reduced pressure of about 100 to 400 microbars preferably about 150 to 300 microbars for about 8 to 12 at a temperature of about -50° C to + 5° C preferably at a temperature of about -25° C to + 5°C.
- Thereafter a secondary drying step may be carried out under nitrogen with a reduced pressure the reduced pressure may be about 40 to 150 preferably about 60 to100 microbars for 3 to 5 hours and at a temperature of about -5° C to + 25° C preferably at a temperature of about + 5° C to + 15° C.
- Removing vacuum by a suitable method known by the skilled practitioner using sterile filtered nitrogen and thereafter the vials may be stoppered under nitrogen and brought out the freeze-drier for crimping.

The freeze-dried preparation according to this process can be re-dissolved with great ease.
The present invention further provides an immediate release formulation (IRF) and/or a continuous release of the active substance over a period of one day to one week when the pharmaceutical formulation is placed in an aqueous physiological environment.
In a particular embodiment of the invention, the immediate release formulation is injectable. This pharmaceutical composition will be used by parenteral way such as subcutaneous, intramuscular, intravenous or infusion route.
As further preferred embodiment the pharmaceutical formulation of the present invention is an immediate release formulation (IRF) administered by parenteral route.
Further according to the present invention the parenteral administration is preferably a subcutaneous administration with daily administration repeated for 7 days and according to patient response repeated treatment several days or weeks, preferably in continous treatment but not limited to this method of administration.
As further object of the invention the pharmaceutical formulations show a high control of the release, expressed by a zero order curve or pseudo zero order curve (zero order kinetics curve means a constant rate of release of the

active substance), and a low $C_{max}$ ($C_{max}$ is a maximum peak observed in plasma concentration of the active substance) therefore provide a control of the initial burst.

The object has been achieved by means of in-vivo and in-vitro tests and stability data provided in the invention.

In addition, various techniques such as radiosterilisation or autoclave sterilisation could be used for the formulations as disclosed in the invention to obtain an aseptic preparation.

The active substances described before, can be used for the treatment and/or prevention of chronic disorders or diseases such as of acromegaly, pituitary adenoma and symptoms associated with neuroendocrine (particularly carcinoid) tumours.

The compound is also indicated for long term treatment of symptomatic, non functional pituitary adenoma.

The following examples serve as illustration of the invention without limiting it.

**Example 1**

**Drawing and Formula of BIM23A760 which corresponds to Dop2-DLys(Dop2)-c(Cys-Tyr-DTrp-Lys-Abu-Cys)-Thr-$NH_2$**

BIM23A760 is a powder product, white to off white color.

Molecular Weight : ~1690 da

Formula : Dop2-DLys(Dop2)-c(Cys-Tyr-DTrp-Lys-Abu-Cys)-Thr-$NH_2$

Molecular structure : $C_{86}H_{116}N_{16}O_{12}S_4$

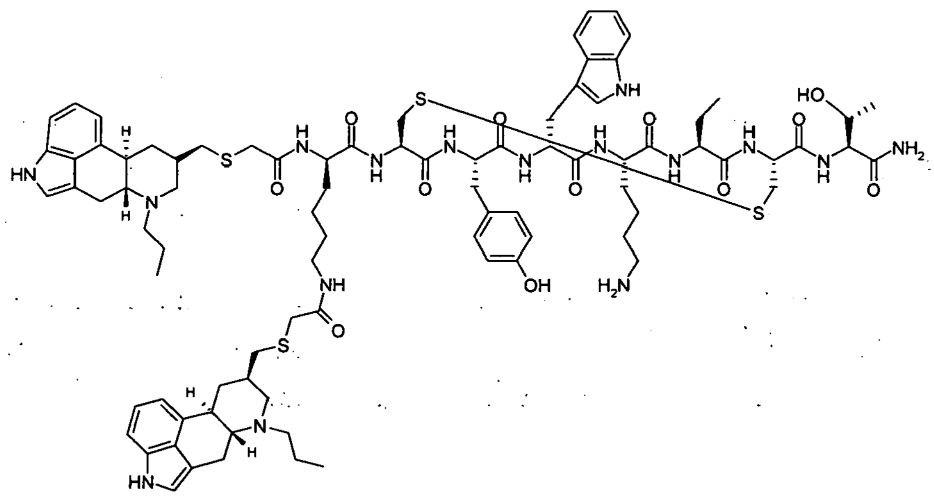

In the formula Dop2-DLys(Dop2)-c(Cys-Tyr-DTrp-Lys-Abu-Cys)-Thr-$NH_2$, by Dop2 it is meant the following formula :

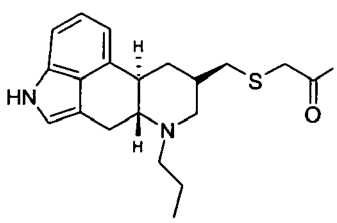

**Example 2**

**Formulation stabilities- method of measurements and results**

Stabilities measurements were performed by means of High Performance Liquid Chromatographic (HPLC) method. The HPLC system a HPLC Waters Alliance 2695 completed by detection system 2487 Dual λ Absorbance Detector, the column used was a $C_{18}$, 250 x 4.6 mm. All details concerning instrumental conditions are gathered in table 4. Elution conditions are gathered in table 5.

Two pharmaceutical forms were studied in parallel for stability assays over six months at + 5° C and + 25° C / 60 % RH (relative humidity) and + 40° C / 75 % RH. Measurements were carried out in parallel with (in either wfi, or 5 % dextrose as solvent) and a lyophilisate (drug substance on its own) or as second variant of formulations with an additional bulking agents such as mannitol, glycine and trehalose) in WFI.

Composition and timing for stabilities durations are reported in the tables 1 and 2.

Table 1

| **Solution form** *per ml* | | |
|---|---|---|
| Formulation 1 | BIM23A760 pure material<br>wfi | 3 % w/v<br>qs |
| Formulation 2 | BIM23A760 pure material<br>Dextrose 5 % in water | 3 % w/v<br>qs |
| **Lyophilisate form** *per vial* | | |
| Formulation 3 | BIM23A760 pure material | 100 % |
| Formulation 4 | BIM23A760 pure material<br>Mannitol apyrogen | 23 % w/w<br>77 % w/w |
| Formulation 5 | BIM23A760 pure material<br>Glycine apyrogen | 23 % w/w<br>77 % w/w |
| Formulation 6 | BIM23A760 pure material<br>Threalose apyrogen | 23 % w/w<br>77 % w/w |

Table 2

| **Temperature storage** | **Time points** |
|---|---|
| + 5° C | T3 month, T6 month |
| + 25° C / 60 % RH | Tzero, T3 month, T6 month |
| + 40° C / 75 % RH | T1 month, T3 month, T6 month |
| Stability results are gathered in table 3 | |

Table 3

| *Lyophilisate* form | +5°C | | | + 25° C / 60 % RH | | | + 40° C / 75 % RH | | |
|---|---|---|---|---|---|---|---|---|---|
| | Δ C (% initial) | P(% a/a) | pH | Δ C (% initial) | P(% a/a) | pH | Δ C (% initial) | P(% a/a) | pH |
| BIM23A760 | | | | | | | | | |
| T0 | / | / | / | 100 % | 95.90 % | 6.8 | / | / | / |
| T1 Month | / | / | / | / | / | / | - 48.0 % | 95.9 % | 6.87 |
| T3 Month | +3.6 % | 96.10% | 6.64 | - 1.2 % | 93.70 % | 6.12 - | 71.0 % | 95.5 % | 5.60 |
| T6 Month | -27.0 % | 96.80% | 5.81 | not analysed | not analysed | 6.63 | not analysed | not analysec | 5.62 |
| BIM23A760 | | | | | | | | | |

(continued)

| *Lyophilisate* form | +5°C | | | + 25° C / 60 % RH | | | + 40° C / 75 % RH | | |
|---|---|---|---|---|---|---|---|---|---|
| | Δ C (% initial) P(% a/a) pH | | | Δ C (% initial) P(% a/a) pH | | | Δ C (% initial) P(% a/a) pH | | |
| / Mannitol | / | / | / | 100 % | 95.80 % | 6.40 | / | / | |
| T0 | / | / | / | / | / | / | - 35.0 % | 95.9% | 6.87 |
| T1 Month | +0.2% | 96.60% | 6.28 | -2.3 % | 92.60 % | 6.12 - | -58.0% | 95.9 % | 5.85 |
| T3 Month | -1.2% | 96.60% | 6.45 | not analysed | not analysed | 6.39 | not analysed | not analysed | 6.51 |
| T6 Month | | | | | | | | | |
| BIM23A760 | | | | | | | | | |
| / Glycine | / | / | | 100 % | 96.50 % | 6.8 | / | / | / |
| T0 | / | / | | / | / | / | -35.0 % | 96.50 % | 5.71 |
| T1 Month | +4.8% | 97.90% | 6.48 - | 35.0 % | *97.00 %* | 6.07 | -67.0 % | 92.70 % | 5.67 |
| T3 Month | -25.60% | 96.80% | 5.70 | not analysed | not analysed | 6.14 | not analysed | not analysed | 5.7.1 |
| T6 Month | | | | | | | | | |
| BIM23A760 | | | | | | | | | |
| / Trehalose | / | / | / | 100 % | 96.00 % | 5.90 | / | / | / |
| T0 | / | / | / | / | / | / | +3.0 % | 95.70% | 6.78 |
| T1 Month | +6.5 % | 97.60% | 5.44 | + 6.8 % | 96.60 % | 6.09 | - 1.1 % | 94.40 % | 6.79 |
| T3 Month | +6.8 % | 96.50% | 5.41 | -1.1 % | 96.00% | 6.06 | -23.74 % | 87.30 % | 6.80 |
| T6 Month | | | | | | | | | |

HPLC Method used for the exploratory stability testingTable 4

| Instrumental conditions | |
|---|---|
| HPLC System | HPLC Waters Alliance 2695 |
| Detection System | 2487 Dual A Absorbance Detector |
| Column (Type, Dimension and packing) | $C_{18}$, 250 x 4.6 mm ID, YMC ODS-AM, 5μm, 12 nm |
| Mobile Phase | A : 0.05M $NaH_2PO_4$ in water, pH adjusted to 3.0 with $H_3PO_4$. |
| | B : 100 % Acetonitrile |
| Flow Rate | 1.0 mL/min |
| Run Time | 50 min |
| Detection (UV,nm) | UV, 220 nm |
| Injection Volume (μl) | 10 μl |
| Target concentration | 0,5 mg / mL |
| Sample preparation diluent | 0,1 N Acetic Acid |
| Column temperature | 30° C |

In table 5 are gathered the elution program, the ratio of each eluants (mobile phase) used for mesurment of formulation stabilities.

Table 5

| Elution Gradient Conditions | | |
|---|---|---|
| Time (min) | Eluant A (%) | Eluant B (%) |
| 0 | 75 | 25 |

(continued)

| Elution Gradient Conditions | | |
|---|---|---|
| Time (min) | Eluant A (%) | Eluant B (%) |
| 30 | 60 | 40 |
| 35 | 40 | 60 |
| 40 | 40 | 60 |
| 41 | 75 | 25 |
| 50 | 75 | 25 |

Standard solution with a concentration of 0.5 mg/ml of BIM 23A760 was prepared by dissolving about 10 mg of active substance BIM23A760 in a flask glass. Thereafter 20 ml of acetic acid 0,1 N were added uder shaking by means of a vortex and if required additionally dissolved in ultrasonic bath in order to otain a total dissolution. Weight of Brut BIM 23A 760 is calculated by means of the following relation :

$$Weigh.of.brut\ BIM23A760 = \frac{10mg\ of\ pure\ BIM23A760}{Potency\ of\ BIM23A760} * 100$$

The dilutions were made by means of a micropipette.

The results showed that the trehalose lyophilisate formulation in comparison to the tested liquid formulation is the most stable form at + 5° C and + 25° C over the first 3 months. After 6 months at + 5° C and + 25° C, the trehalose-based lyophilisate remains the most stable in terms of cake appearance, reconstitution, pH, chromatographic profile and assay. The lyophilisate and the liquid formulations are stable for at least 24 months at ± 5° C.

The trehalose-based lyophilisate ensures solubilisation, and stability of the active drug over time. Mannitol and Glycine were also experimented on freeze dried and stored at + 25° C or + 40° C over 3 or 6 months.

Saccharides or di-sacharrides have been tested (xylitol, maltose, lactose, galactose, dextran) to check the compound stability in such formulation.

Nevertheless based on the results trehalose ensures and improves BIM23A760 physico-chemical stability. Trehalose allows to protect and stabilize the active drug in the freeze dried formulations.

The molecule BIM23A760 for subcutaneous administration is supplied as a "powder for solution for infusion" in clear vials. The finished drug product is reconstituted either with water for injections or dextrose 5 % for infusion.

Example 3

Example of a freeze-dried formulation

| Material | Doses |
|---|---|
| BIM23A760 pur | 5.0 mg |
| Trehalose | 17.0 mg |
| Nitrogen | vial closed under nitrogen |

The lyophilisate is available in 11 mL Type I glass vial and stoppered with a grey chlorobutyl stopper under nitrogen.

Currently the lyophilisate is at least stable 24 months at + 5° C.

The lyophilisate is to be reconstituted either with an adequate volume of water for injection or dextrose 5 % (= 1 ml) according to the required clinical dosage. The volume of reconstitution is from 1 ml to 10 ml.

Example 4

Process manufacturing of the freeze-dried formulation

BIM23A760 lyophilisate 5.0 mg is manufactured by dissolving the active drug and the ingredients in water for injections under stirring. This solution is then bubbled under nitrogen sterilised by sterile filtration through 0.22 micrometer PVDF membrane filters and aseptically filled into sterile vials. The theoretical filling is 1.0 g ± 3 % of solution.

Filled vials are pre-stoppered and transferred into the freeze-drier. Freeze-drying conditions used (freeze drying parameters monitoring) are provided as an example:

Freezing : 3 hours 30 min at -25° C

Primary desiccation under nitrogen and vacuum 150 to 300 microbars : 10 hours from -25° C to + 5° C.

Secondary desiccation under nitrogen and vacuum 60 to100 microbars : 4 hours 30 min from + 5° C to + 15° C.

At the end of freeze-drying cycle the vacuum is released by means of sterile filtered nitrogen. Vials are stoppered inside the freeze-dryer, by tray pressure. Stoppered vials are brought out of the freeze-dryer for crimping.

Stoppered vials are automatically crimped. Then vials are externally washed with single use wipes. Bulk product is 100 % visually inspected then stored at + 5° C ± 3° C in sealed containers.

Process flow chart

Table 6

| Step | Operations |
|---|---|
| A | Automatic Vials washing<br>OVEN Depyrogeneisation<br>Stoppers Caps, Filters, ... autoclave<br>Active and ingredients weighing<br>wfi weighing<br>dissolution under stirring / Bubbling |
| B | Sterilizing Filtration |
| C | Automatic aseptic filling into 11 ml glass vial. Vial pre stoppering |
| D | Freeze dryer sterilisation<br>Freeze drying<br>Vial stoppering under sterile nitrogen |
| E | Vial crimping<br>External cleaning and inspection<br>Labelling and bulk packing |
| F | Storage at + 5°C ± 3 °C |

Examples 5

Pharmacokinetics data and methods used for providing the data

Reconstituted Lyophilisate (+ 1 ml of wfi) has been administered on animals by subcutaneous route, in order to evaluate the Pk, on Sprague Dawleys rats versus liquid formulation used for preclinical development (2 % Solutol Formulation in wfi).

Two series of diluted solution were made, one serie with solutol 2 % and the second with trealose. Different

dilutions were prepared with 0.1 mg/ml, 1 mg/ml and 10 mg/ml of BIM23A760. Comparatives data are disclosed on figures 1a and 1b.

Preliminary pharmacokinetic study after single subcutaneous injection of BIM23A760 at different concentrations to Sprague-Dawley Rats are disclosed in table 7.

Table 7

| Formulation | Group ID | Actual Dose *mq/kg* | Dose Concentration *mg/mL* | $t_{max}$ *h* | $C_{max}$ *ng/mL* | $C_{max}$/D - | $AUC_t$ *(ng·h/mL)* | $AUC_{al}$ *(ng·h/mL)* | $AUC_t$/D - | $MRT_t$ *h* |
|---|---|---|---|---|---|---|---|---|---|---|
| 2 % Solutol in wfi | 1 | 0.183 | 0.1 | 8 | 6.896 | 37.68 | 127.5 | 133.9 | 696.7 | 14.06 |
| | 2 | 0.181 | 1 | 0.25 | 13.66 | 75.47 | 52.62 | 61.54 | 290.7 | 15.54 |
| | 3 | 0.179 | 10 | 0.25 | 66.16 | 369.6 | 124.2 | 132.4 | 693.7 | 12.64 |
| | 4 | 0.896 | 10 | 0.25 | 127.8 | 142.6 | 582.2 | 592.4 | 649.8 | 18.12 |
| | 5 | 1.028 | 25 | 0.25 | 215.1 | 209.2 | 1072 | 1085 | 1043 | 16.42 |
| Trehalose Formulation | 6 | 0.191 | 0.1 | 0.25 | 125.8 | 658.6 | 133.1 | 139.2 | 697.0 | 8.013 |
| | 7 | 0.184 | 1 | 0.25 | 65.55 | 356.3 | 95.81 | 108.7 | 520.7 | 12.30 |
| | 8 | 0.187 | 10 | 1 | 46.90 | 250.8 | 241.4 | 248.9 | 1290.8 | 13.55 |
| | 9 | 0.933 | 10 | 1 | 70.52 | 75.58 | 705.2 | 719.6 | 755.8 | 24.24 |
| | 10 | 1.072 | 25 | 1 | 97.83 | 91.3 | 723.9 | 731.2 | 675.3 | 25.62 |

## Claims

1. A stable solid pharmaceutical formulation at temperatures that can range from +5° C to +40° C obtenable by lyophilisation comprising a chimeric molecule and at least one sugar or a polyols or a mixture thereof.

2. The formulation according to claim 1 **characterized in that** the chimeric molecule is BIM23A760 or a dopamine derivatives.

3. The formulation according to claim 1 or 2 **characterized in that** the sugar of the stable solid pharmaceutical formulation is selected from saccharides or disaccharides or polyols which include xylitol, maltose, lactose, galactose, mannitol, sorbitol, dextran and threalose or a mixture thereof preferably xylitol, mannitol or threalose.

4. A process for the preparation of the pharmaceutical formulation of claim 1 comprising :

   (a) Introduction and weighing of the active substance and excipients.
   (b) Dissolution of compounds by means of water under stirring / bubbling under nitrogen.
   (c) Filtration.
   (d) Filling into a vial and pre-stoppering.
   (e) Pre frozen step and freeze-drying step under vacuum.
   (f) Releasing vacuum and stoppering under controlled athmosphere

5. The process according to claim 4 is **characterized in that** the pre frozen temperature of step (e) is comprised from about -20° C to -50° C prefably from about -20° C to -30° C more preferably at about -25° C to -30° C under inert conditions.

6. The process according to claim 4 or 5 **characterized in that** release of vacuum is made under controlled athmosphere consisting in bubbling with nitrogen.

7. A stable liquid pharmaceutical formulation at temperatures that can range from +5° C to +40° C obtainable according to steps (a) to (d) of the process of claim 4 comprising a chimeric molecule and at least one sugar or a polyols or a mixture thereof and optionally containing an isotonic agent and/or a buffer.

8. The stable liquid pharmaceutical formulation according to claim 7 **characterized in that** the chimeric molecule is BIM23A760 or a dopamine derivatives.

9. The stable liquid pharmaceutical formulation according to claim 7 or 8 is **characterized in that** the isotonic agent is selected from propylene glycol, glycerol, sodium chloride or potassium chloride.

10. The stable liquid pharmaceutical formulation according to claims 7 to 9 is **characterized in that** the sugar is selected from saccharides or disaccharides or polyols which include xylitol, maltose, lactose, galactose, mannitol, sorbitol, dextran and threalose or a mixture thereof preferably threalose, xylitol or mannitol.

11. Use of BIM BIM 23A760 in freeze dried formulation prepared according to the process of claims 4 to 6 for the treatment and/or prevention of chronic disorders or diseases such as acromegaly, pituitary adenoma and symptoms associated with neuroendocrine (particularly carcinoid) tumours.

12. Use of BIM 23A760 in liquid formulation prepared according to the process of claim 4 steps (a) to (d), for the treatment and/or prevention of chronic disorders or diseases such as acromegaly, pituitary adenoma and symptoms associated with neuroendocrine (particularly carcinoid) tumours.

13. The invention as herein before defined.

**Comparative Pk profile after Single Subcutaneous injection at different concentrations in Rats 2 % Solutol in wfi Fomulation versus Trehalose Formulation**

Figure 1a

Pharmacokinetic profile of 2 % Solutol Solution Formulation in Rats - Dose 0.2 mg/kg

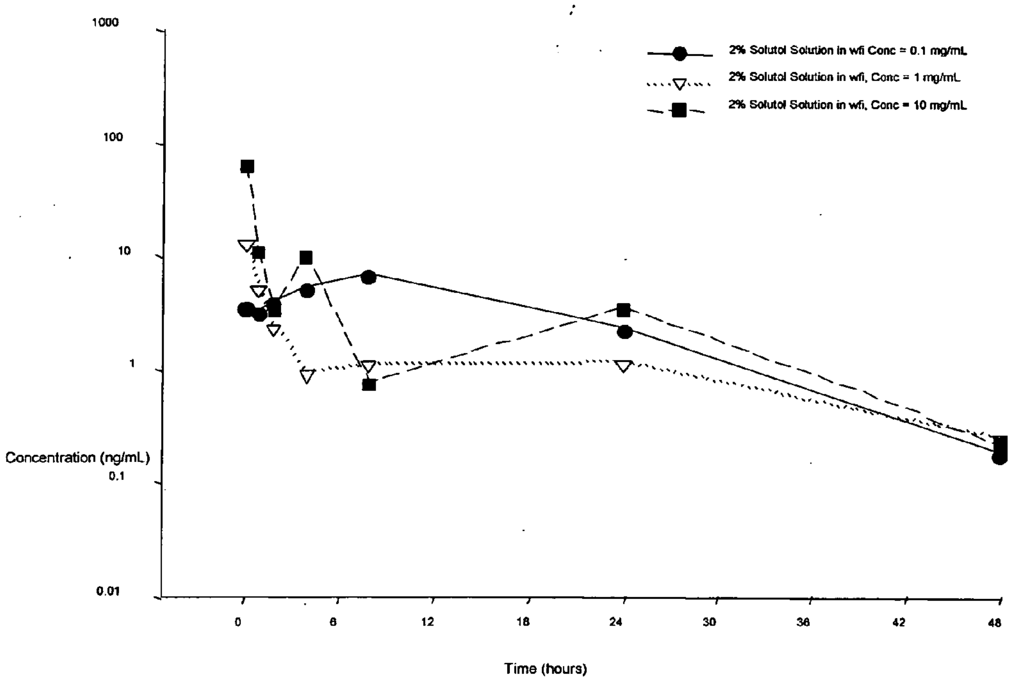


Figure 1b

Pharmacokinetic profile of Trehalose formulation in Rats - Dose = 0.2 mg/kg

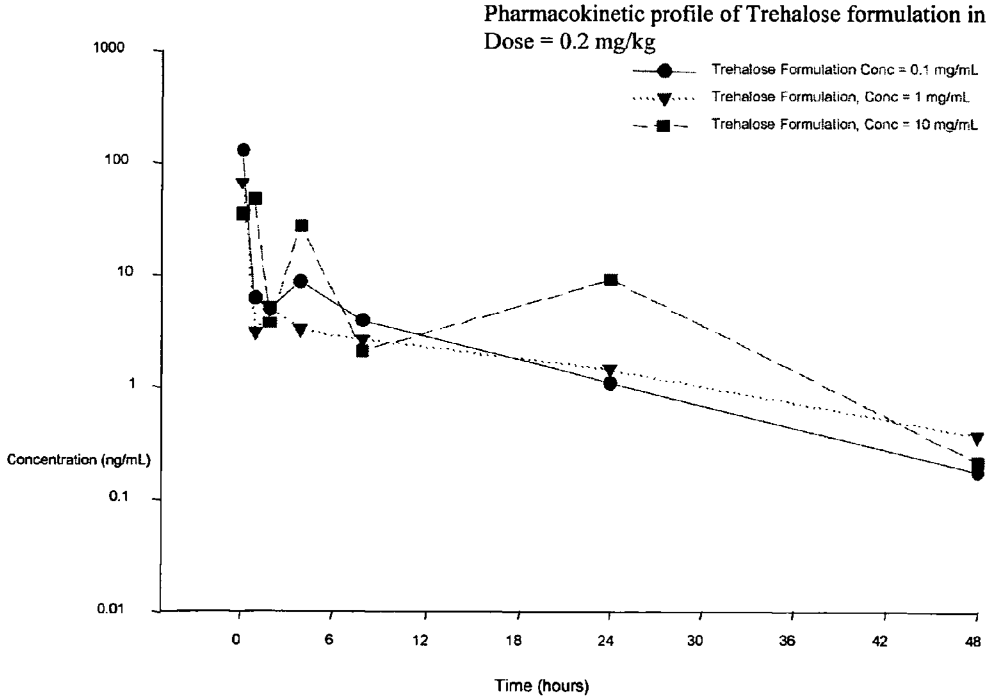

Figure 2

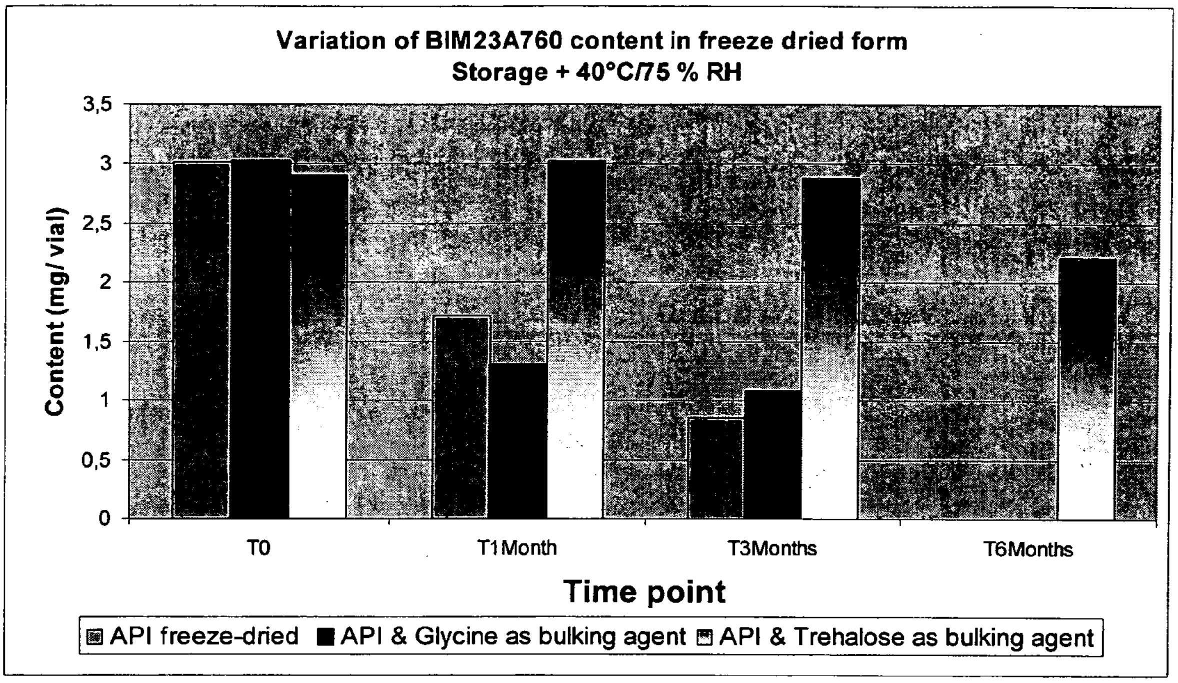
Variation of BIM23A760 content in freeze dried form
Storage + 40°C/75 % RH
Content (mg/ vial)
3,5
3
2,5
2
1,5
1
0,5
0
T0
T1Month
T3Months
T6Months
Time point
API freeze-dried
API & Glycine as bulking agent
API & Trehalose as bulking agent

European Patent Office

# EUROPEAN SEARCH REPORT

Application Number
EP 08 29 0330

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 99/44604 A (SHIONOGI & CO [JP]; CONFER WILLIAM LESTER [US]; TAI HIDEAKI [JP]) 10 September 1999 (1999-09-10)<br>* claims 1-27 * | 1-13 | INV.<br>A61K9/19<br>A61K38/00<br>A61P35/00 |
| X | EP 1 762 249 A (ALTANA PHARMA AG [DE]) 14 March 2007 (2007-03-14)<br>* examples 1-4 *<br>* claims 1-21 * | 1-13 | |
| X | WO 2005/102274 A (PFIZER PROD INC [US]; PIKAL MICHAEL J [US]; REDDY RENUKA DEVI [US]; SH) 3 November 2005 (2005-11-03)<br>* example 1 * | 1-13 | |
| X | EP 0 443 471 A (YOSHITOMI PHARMACEUTICAL [JP]; YAMASA SHOYU KK [JP]) 28 August 1991 (1991-08-28)<br>* example 3 * | 1-13 | |
| X | WO 02/41919 A (BYK GULDEN LOMBERG CHEM FAB [DE]; DIETRICH RANGO [DE]; LINDER RUDOLF [) 30 May 2002 (2002-05-30)<br>* examples 1-4 * | 1-13 | TECHNICAL FIELDS SEARCHED (IPC)<br>A61K<br>A61P |
| X | GB 2 248 550 A (SANDOZ LTD [CH]) 15 April 1992 (1992-04-15)<br>* examples 1-14 * | 1-13 | |
| X | WO 03/097101 A (NOVUSPHARMA SPA [IT]; BERNAREGGI ALBERTO [IT]; LIVI VALERIA [IT]) 27 November 2003 (2003-11-27)<br>* claims 1-11 * | 1-13 | |

-/--

The present search report has been drawn up for all claims

| Place of search | Date of completion of the search | Examiner |
|---|---|---|
| Munich | 30 July 2008 | Albayrak, Timur |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)
9

European Patent Office

# EUROPEAN SEARCH REPORT

Application Number
EP 08 29 0330

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | SAVEANU ALEXANDRU ET AL: "SOMATOSTATIN AND DOPAMINE-SOMATOSTATIN MULTIPLE LIGANDS DIRECTED TOWARDS SOMATOSTATIN AND DOPAMINE RECEPTORS IN PITUITARY ADENOMAS" NEUROENDOCRINOLOGY, S.KARGER, BASEL, CH, vol. 83, no. 3-4, 1 January 2006 (2006-01-01), pages 258-263, XP009085931 ISSN: 0028-3835 * the whole document * ----- | 11-13 | |
| Y | ANDERSEN M: "The role of lanreotide Autogel(R) in the treatment of acromegaly" EXPERT REVIEW OF ENDOCRINOLOGY AND METABOLISM 200707 GB, vol. 2, no. 4, July 2007 (2007-07), pages 433-441, XP002490121 ISSN: 1744-6651 * the whole document * ----- | 11-13 | |
| Y | JAQUET P ET AL: "BIM-23A760, a chimeric molecule directed towards somatostatin and dopamine receptors, vs universal somatostatin receptors ligands in GH-secreting pituitary adenomas partial responders to octreotide." JOURNAL OF ENDOCRINOLOGICAL INVESTIGATION 2005, vol. 28, no. 11 Suppl International, 2005, pages 21-27, XP009103539 ISSN: 0391-4097 * the whole document * ----- | 11-13 | TECHNICAL FIELDS SEARCHED (IPC) |

The present search report has been drawn up for all claims

| Place of search | Date of completion of the search | Examiner |
|---|---|---|
| Munich | 30 July 2008 | Albayrak, Timur |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

9

## ANNEX TO THE EUROPEAN SEARCH REPORT ON EUROPEAN PATENT APPLICATION NO. EP 08 29 0330

This annex lists the patent family members relating to the patent documents cited in the above-mentioned European search report.
The members are as contained in the European Patent Office EDP file on
The European Patent Office is in no way liable for these particulars which are merely given for the purpose of information.

30-07-2008

| Patent document cited in search report | Publication date | Patent family member(s) | Publication date |
|---|---|---|---|
| WO 9944604 A | 10-09-1999 | AT 310513 T | 15-12-2005 |
| | | AU 757002 B2 | 30-01-2003 |
| | | AU 2799899 A | 20-09-1999 |
| | | BR 9908479 A | 05-12-2000 |
| | | CA 2322796 A1 | 10-09-1999 |
| | | CN 1299277 A | 13-06-2001 |
| | | DE 69928500 D1 | 29-12-2005 |
| | | DE 69928500 T2 | 03-08-2006 |
| | | EA 3864 B1 | 30-10-2003 |
| | | EP 1058547 A1 | 13-12-2000 |
| | | ES 2253877 T3 | 01-06-2006 |
| | | HU 0102812 A2 | 28-12-2001 |
| | | ID 27487 A | 12-04-2001 |
| | | JP 2002505282 T | 19-02-2002 |
| | | JP 2006096761 A | 13-04-2006 |
| | | NO 20004306 A | 10-10-2000 |
| | | NZ 506578 A | 26-09-2003 |
| | | PL 342822 A1 | 02-07-2001 |
| | | TR 200002543 T2 | 21-11-2000 |
| | | TW 570795 B | 11-01-2004 |
| EP 1762249 A | 14-03-2007 | EP 1339430 A1 | 03-09-2003 |
| | | SI 1339430 T1 | 30-06-2008 |
| WO 2005102274 A | 03-11-2005 | AT 374624 T | 15-10-2007 |
| | | BR PI0509992 A | 16-10-2007 |
| | | CA 2563665 A1 | 03-11-2005 |
| | | DE 602005002742 T2 | 24-01-2008 |
| | | EP 1740214 A2 | 10-01-2007 |
| | | ES 2292122 T3 | 01-03-2008 |
| | | JP 2007533724 T | 22-11-2007 |
| | | US 2008096858 A1 | 24-04-2008 |
| EP 0443471 A | 28-08-1991 | AT 118012 T | 15-02-1995 |
| | | CA 2036507 A1 | 20-08-1991 |
| | | DE 69107098 D1 | 16-03-1995 |
| | | DK 443471 T3 | 26-06-1995 |
| | | ES 2067780 T3 | 01-04-1995 |
| | | JP 1888153 C | 07-12-1994 |
| | | JP 3240794 A | 28-10-1991 |
| | | JP 6015557 B | 02-03-1994 |
| | | US 5183882 A | 02-02-1993 |
| WO 0241919 A | 30-05-2002 | AT 381946 T | 15-01-2008 |
| | | AU 1604202 A | 03-06-2002 |
| | | AU 2002216042 B2 | 03-08-2006 |

EPO FORM P0459

For more details about this annex : see Official Journal of the European Patent Office, No. 12/82

## ANNEX TO THE EUROPEAN SEARCH REPORT ON EUROPEAN PATENT APPLICATION NO. EP 08 29 0330

This annex lists the patent family members relating to the patent documents cited in the above-mentioned European search report.
The members are as contained in the European Patent Office EDP file on
The European Patent Office is in no way liable for these particulars which are merely given for the purpose of information.

30-07-2008

| Patent document cited in search report | Publication date | Patent family member(s) | Publication date |
|---|---|---|---|
| WO 0241919 A | | BG 107828 A | 30-07-2004 |
| | | BR 0115483 A | 21-10-2003 |
| | | CA 2428870 A1 | 30-05-2002 |
| | | CN 1476335 A | 18-02-2004 |
| | | CZ 20031416 A3 | 17-09-2003 |
| | | DK 1339430 T3 | 13-05-2008 |
| | | EE 200300182 A | 16-06-2003 |
| | | ES 2298295 T3 | 16-05-2008 |
| | | HK 1060848 A1 | 24-11-2006 |
| | | HR 20030410 A2 | 30-04-2005 |
| | | HU 0303278 A2 | 28-01-2004 |
| | | IS 6820 A | 19-05-2003 |
| | | JP 2004513970 T | 13-05-2004 |
| | | KR 20080003010 A | 04-01-2008 |
| | | MX PA03004548 A | 10-09-2003 |
| | | NO 20032303 A | 21-05-2003 |
| | | NZ 525911 A | 26-11-2004 |
| | | PL 361118 A1 | 20-09-2004 |
| | | SK 6062003 A3 | 07-10-2003 |
| | | TW 284041 B | 21-07-2007 |
| | | UA 80961 C2 | 26-11-2007 |
| | | US 2003003058 A1 | 02-01-2003 |
| | | YU 37703 A | 25-05-2006 |
| | | ZA 200303593 A | 23-04-2004 |
| GB 2248550 A | 15-04-1992 | AU 8464791 A | 26-03-1992 |
| | | CA 2051721 A1 | 21-03-1992 |
| | | CH 683749 A5 | 13-05-1994 |
| | | CS 9102854 A3 | 15-04-1992 |
| | | DE 4131232 A1 | 26-03-1992 |
| | | EP 0490806 A2 | 17-06-1992 |
| | | FI 914398 A | 21-03-1992 |
| | | FR 2666987 A1 | 27-03-1992 |
| | | FR 2681326 A1 | 19-03-1993 |
| | | IE 913296 A1 | 25-02-1992 |
| | | IT 1255258 B | 20-10-1995 |
| | | JP 4247034 A | 03-09-1992 |
| | | LU 88006 A1 | 15-04-1993 |
| | | MX 9101175 A1 | 04-05-1992 |
| | | PT 99007 A | 31-08-1992 |
| | | ZA 9107528 A | 22-03-1993 |
| WO 03097101 A | 27-11-2003 | AT 381944 T | 15-01-2008 |
| | | AU 2003240613 A1 | 02-12-2003 |
| | | CA 2486001 A1 | 27-11-2003 |

EPO FORM P0459

For more details about this annex : see Official Journal of the European Patent Office, No. 12/82

## ANNEX TO THE EUROPEAN SEARCH REPORT ON EUROPEAN PATENT APPLICATION NO. EP 08 29 0330

This annex lists the patent family members relating to the patent documents cited in the above-mentioned European search report.
The members are as contained in the European Patent Office EDP file on
The European Patent Office is in no way liable for these particulars which are merely given for the purpose of information.

30-07-2008

| Patent document cited in search report | Publication date | Patent family member(s) | Publication date |
|---|---|---|---|
| | | EP 1503797 A1 | 09-02-2005 |
| WO 03097101 A | | ES 2298521 T3 | 16-05-2008 |
| | | IT MI20021040 A1 | 17-11-2003 |
| | | JP 2005530792 T | 13-10-2005 |
| | | MX PA04011348 A | 15-08-2005 |
| | | US 2006199831 A1 | 07-09-2006 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 0056365 A

### Non-patent literature cited in the description


- Stabilisation of Octastation, a somatostatin analogue. Preparation of freeze-dried products for parenteral injection. **H. Pourrat.** Biological and Pharmaceutical Bulletin. scientific publication, vol. 18, 766-771
- Efficacy of chimeric molecules directed towards multiple somatostatin and dopamine receptors on inhibition of GH and prolactin secretion from GH-secreting pituitary adenomas classified as partially responsive to somatostatin analog therapy. **P Jaquet.** European Journal of Endocrinology. scientific publication, vol. 153, 135-141